# EUROPEAN PATENT APPLICATION

(11) **EP 2 993 237 A2**
(43) Date of publication of application: **09.03.2016**
(21) Application number: 14733740.6
(22) Date of filing: 30.04.2014
(51) Int. Cl.: C12Q 1/68

(54) **PEPTIDE NUCLEIC ACID (PNA) PROBE, KIT AND METHOD FOR DETECTING AND/OR QUANTIFYING ESCHERICHIA COLI 0157 : H7**

(30) Priority: 30.04.2013 PT 10691613
(71) Applicant: Universidade do Minho, 4704-553 Braga (PT); Universidade do Porto, 4099-002 Porto (PT); Biomode - Biomolecular Determination, S.A., 4806-909 Guimarães (PT)
(72) Inventor: FERNANDES ALMEIDA, Carina Manuela, Vila Verde, Moure Moure (PT); DE SOUSA, José Mário, 4905-043 Barcelos (PT); ALVES ROCHA, Rui Jorge, 4805-373 Guimarães (PT); MACIEIRA CERQUEIRA, Laura Isabel, 4715-288 Braga (PT); DA COSTA VIEIRA, Maria João Lopes, 4710-358 Braga (PT); DE OLIVEIRA AZEVEDO, Nuno Filipe Ribeiro Pinto, 4710-504 Braga (PT)
(74) Representative: Bairrão, Isabel
(86) International application number: PCT/PT2014/000023
(87) International publication number: WO 2014/178741

(57) **Abstract**

PNA is a synthetic molecule analogue to DNA that, due to its physicochemical properties, allows a faster analysis with higher sensitivity and specificity than the DNA probes. These probes are combined with fluorescence *in situ* hybridization (FISH), a molecular biology technique that allows the direct visualization of the microorganisms in the sample. The combination of these two technologies rendered the FISH procedure to be faster, simpler and more efficient.

This method can be applied to a great variety of samples such as food, blood, biopsies, feces, water and other clinical, environmental or agriculture and food industry samples.

The present invention also includes the development of the kit of detection and respective procedure for the specific identification of *Escherichia coli* O157:H7 using the above referred sample types.

## Description

### Field of the invention

This invention relates to a process for the detection of microorganisms relevant for clinical and food safety. For that purpose, a PNA probe for the detection of *Escherichia coli* 0157:H7 serotype was developed.

In addition to the probe, the present invention included the PNA-FISH procedure and its application to a kit for the detection and/or quantification of *Escherichia coli* 0157:H7, which can be used in the food and clinical fields.

### Background of the invention:

*Escherichia coli* species includes a genetically heterogeneous group of bacteria which are part of the normal microflora of the intestinal tract of humans and animals (Gyles, 2007). These bacteria are typically nonpathogens, but a considerable number of strains are pathogenic. Pathogenic strains of *E. coli* are classified according to their specific virulence factors, their pathogenicity and their specific strain. There are 6 classes of pathogenic *E*. *coli*, enterotoxigenic *E. coli* (ETEC), enteropathogenic *E*. *coli* (EPEC), enteroaggregative *E*. *coli* (EAEC), enteroinvasive *E. coli* (EIEC), the diffusely adherent *E*. *coli* (DAEC) and the enterohemorrhagic *E. coli* (EHEC) (Sheibani, 2007). Among pathogenic *E*. *coli,* enterohemorrhagic *E*. *coli* (EHEC) are perhaps the most important because of its virulence and its association with life-threatening complications (Sheibani, 2007). Within EHEC group, *E*. *coli* serotype 0157:H7 (the serotype is based on the 0 (Ohne) antigen, determined by the polysaccharide portion of cell wall lipopolysaccharide (LPS) and the H (Haunch) antigen due to flagella protein) is the most commonly isolated (Gyles, 2007).

The infectious dose of *E. coli* O157:H7 is reported to be very low - around 1-100 CFU/mL - lower than most enteric pathogens (Robinson and McKillip, 2010). The virulence of this microorganism is due to 3 major factors. The first and by far the most critical, is the production of one or both phage-encoded Shiga toxins (Stxs), called Stx1 and Stx2. These Stxs are among the most potent cytotoxins currently known to affect eukaryotic cells (Robinson and McKillip, 2010). Although the type of Stx produced directly influences the severity of disease, the production of Stxs alone is insufficient for *E. coli* 0157:H7 became pathogenic. Instead, the assistance of the locus of enterocyte effacement (a bacterial pathogenicity island, that encodes a battery of specialized virulence factors) and the pO157 plasmid (encodes as type II secretion system, an enterohemolysin, a serine-protease, a catalase-peroxidase, a lymphocyte inhibitor, and potential adhesions, among others) is required (Robinson and McKillip, 2010).

The effects of an EHEC infection are quite diverse, ranging from asymptomatic to lethal. The disease is self-limiting and the symptoms resolve itself after one week. In severe cases the patients can develop Hemolytic-Uremic Syndrome (HUS), Thrombocytopenic Thrombotic Purpura (TTP) or even die (Robinson and McKillip, 2010). HUS targets renal endothelial cells and can le ad to acute renal failure (Robinson and McKillip, 2010). HUS have considerably high mortality rate and in case of survival, patients are left with permanent renal consequences. In addition to typical HUS symptoms, TTP have associated neurological symptoms like severe headaches, convulsions, lethargy and encephalopathy (Robinson and McKillip, 2010). For the *E. coli* O157:H7 outbreaks reported in the USA, 25% of affected persons were hospitalized, 5 - 10% developed HUS or TTP and 1% died (Pennington, 2010).

Regarding environmental reservoirs, cattle serve as the primary and natural reservoir of *E*. *coli* 0157:H7 and it is estimated that 10 - 80% of all cattle are colonized (Yoon and Hovde, 2008). Other animals such as goats, sheep and pigs may be carriers as well. Results from a study of 90 outbreaks confirmed microbiologically (between 1982 and 2006) showed that the source of transmission to humans was 42.2% associated to food, 12.2% to dairy products, 7.8% to animal contact, 6 - 7% to water, 2.2% to environmental and unknown in 28.9% of the outbreaks (Pennington, 2010).

Before food products are shipped to consumer markets, they must be inspected for contamination during quality control measures. Because of the importance of quality control, the methods used to detect bacterial contaminants must be rapid, sensitive and reliable as well as versatile in order to accommodate the dynamic needs of the food processing plant and that can substantially cut down the time, labor and overall cost of detection process.

Testing for sorbitol fermentation has been suggested as simple manner to screen *E*. *coli* 0157:H7, because it lacks the β-glucorunidase enzyme. Most existing culture methods were developed based on this feature. The bacteria inability to ferment rhamanose and tolerance to tellurite, have been also explored (Raji *et al.*, 2003). These traditional plating techniques remain an integral aspect of quality control during food processing because they are both cost-effective and technically simple, with high level of accuracy and sensitivity. These plating techniques however, are very time consuming, laborious and fail to detect *E. coli* 0157:H7 which ferment sorbitol and are susceptible to tellurite (Raji *et al.,* 2003). They also fail to detect pathogens in low numbers (< 200 CFU/g sample). Moreover, culture methods such as ISO 16654, usually include an agglutination assay (detecting 0157 or H7 antigen) that are not specific, since the 0157 and H7 antigen is present in other *Escherichia coli* species. These antibodies can also cross-react with other *E. coli* serotypes, *Escherichia* species and other members of the *Enterobacteriaceae* family (Raji *et al.,* 2003).

FISH is a molecular assay widely applied in identification of microorganisms. This method is based on the specific binding of small oligonucleotides (probes) to particular rRNA regions due to its high cellular abundance, universal distribution and use as a phylogenetic marker. The probe is coupled to a fluorochrome and after the hybridization a fluorescence signal can be detected due to the high rRNA copy number within the cell. More recently, peptide nucleic acid probes (PNA) have been developed for microbial detection (Cerqueira *et al.,* 2008). These molecules mimic DNA and are capable of hybridizing specifically with complementary nucleic acids obeying to the Watson-Crick rules. The establish bond is stronger since in the PNA molecule, have a neutral repeated N-(2-aminoethil) glycin unit instead of the negative charged sugar-phosphate backbone. The adequate use of this molecule in FISH technology has become the procedure more robust, quicker and more efficient, which allowed the development of several PNA-FISH methods for the detection of pathogenic organisms (Cerqueira *et al.,* 2008).

In here, we have developed a new PNA FISH based method for the specific detection of *E*. *coli* 0157:H7 in food and or clinical samples. This is the first PNA-FISH method developed for detecting a specific serotype.

### Summary of the invention

The present invention refers to the development of a Peptide Nucleic Acid (PNA) probe and method thereof for the detection of *Escherichia coli* 0157:H7 serotype (that is, identification and quantification).

The probe described in the present invention recognizes the 23S rRNA of the microorganism in question or the genomic sequences corresponding to the rRNA mentioned. The PNA probes have physiochemical characteristics that are inherent to its structure and when they are applied to a FISH-based method, allow a faster, more robust and more specific analysis than using a DNA probes.

One of the advantages of this method is that the probe works robustly in a wide variety of biological samples, which usually does not happen with the other detection molecular methods.

Another relevant aspect is the time required for detection. The method here developed matches the best times reported for the remaining molecular methods, even when the type of sample requires an enrichment step prior to the analysis. The rapidity and the reliability of the method can determine the appropriate and timely treatment of a contamination and/or infection for both clinical or food security perspectives.

Another aspect of the present invention is related to the development of a kit based on the application of this blocker probe to fluorescence *in situ* hybridization (FISH), allowing the specific detection of *E. coli* 0157:H7 in a broad range of biological samples, in a prompt and simple way.

In a preferred embodiment of the present invention, the PNA probe here described allow the detection of the target sequence in rRNA, in rDNA or in complementary sequences of the rRNA of *E*. *coli* 0157.

One of the embodiments of the present invention is the description of a PNA probe used to the detection and/or quantification of *E. coli* 0157 characterized in that it has at least 86% of similarity to the sequence SEG ID No. 1 -5'- CAA CAC ACA GTG TC -3', preferably 87%, 88%, 89%, 90%, 91%, 92%, 93%, 94%, 95%, 96%, 97%, 98%, 99%, 100% of similarity to the sequence SEG ID No. 1 -5'- CAA CAC ACA GTG TC -3.

In a more preferable embodiment of the present invention, the previously described sequence is linked to at least one type of detectable fraction. The type of detectable fraction to be used may be selected from one of the following groups: a conjugate, a branched detection system, a chromophore, a fluorophore, radioisotope, an enzyme, a hapten or a luminescent compound, among others.

In an even more preferable embodiment, the fluorophore group can be at least one of the following: Alexa series fluorophores, cyanines, 5- (and -6) Carboxy-2', 7'-dichlorofluorescein, 5-ROX (5 -carboxy-X-rhodamine, triethylammonium salt), among others.

It is still the subject of the present invention a kit for the specific detection of the presence or absence and/or quantification of *E. coli* 0157 in biological samples.

In a more preferable embodiment of the present invention, the kit may additionally present at least one of the following solutions: a fixation solution, a hybridization solution and a washing solution.

In yet another preferred embodiment of the present invention, the fixation solution can comprise paraformaldehyde and ethanol, namely 2-8% (weight/vol) of paraformaldehyde and 25-90% (vol/vol) of ethanol and/or hybridization solution may comprise formamide.

It is still the object of the present invention the description of a method for the specific detection of *E*. *coli* 0157 or for the detection of *E. coli* 0157 in biological samples, which uses the PNA probe mentioned earlier and which comprises the following steps:
- contact of the PNA probe with biological samples;
- hybridization of the detecting probe with the target sequence of the microorganisms present within the biological samples;
- detection of the hybridization as an indication of the mentioned detection and quantification in the biological samples, the hybridization may be preferably carried on by fluorescence.

The biological samples can be taken from food, feces, blood, air, water or biopsies among others.

It is still the object of the present invention the use of the PNA probe described earlier, the use of the kit described earlier and the methodology to be applied in a methodology of detection of *E. coli* 0157, or detection of *E*. *coli* in biological samples.

### General description of the invention

The present invention comprises the PNA probe, reagents, methods and a kit intended for the detection or quantification of *E. coli* 0157 strains.

The PNA probe herewith described allows the specific detection of the *E*. *coli* 0157 through the bonding to rRNA, genomic sequences corresponding to rRNA (r), or their complementary sequences.

The higher specificity of the PNA probes (in relation to DNA probes) allows a better discrimination between related nucleotide sequences. This has particular relevance to this probe since there are some phylogenetically related microorganisms to *E. coli* 0157 that present only one mismatch (nucleotide at position 8 of the probe described in this invention) within the selected target region. Some examples of this situation are the *Escherichia coli* non-O157:H7 strains.

The PNA probe described in this invention has 14 nucleotides with the following nucleotidic sequence:
SEQ ID No. 1 - 5'- CAA CAC ACA GTG TC -3'.
However, the probe to be used can present at least 86% of identity to the sequence mentioned above.

This probe is applied to the analysis by fluorescence in *situ* hybridization (FISH), which, in the case of *E. coli* 0157 positive samples, results in the emission of detectable fluorescent signal either through fluorescence microscopy or through flow cytometry.

The development of the new PNA-FISH probe was carried out empirically using specific software. The selection of the probe sequence was initially performed by aligning rDNA sequences of the target microorganism, with sequences of related microorganisms. This allowed the identification of potentially useful regions, which will be then evaluated based on other parameters such as specificity, hybridization temperature, percentage of guanine/cytosine, bond free energy and secondary structure.

After the probe design and synthesis, the three steps of FISH procedure, fixation/permeabilization, hybridization and wash, have to be developed and optimized for the selected probe. This process usually involves the following parameters: temperature, concentration of formamide and ethanol, and hybridization and washing times. It is important to notice that due to the process complexity and the large number of variables, is not always possible to develop a method for each sequence and as such, several alternatives and sequences are often tested.

A well-succeeded hybridization afterwards allows inferring about the presence/absence and even the concentration of a microorganism by fluorescence microscopy, flow cytometry or real time PCR. The detected fluorescent signal is generally the result of the specific binding of the small probes to tens or hundreds of rRNA copies present in the bacteria cytoplasm. That detectable fraction of the probe, which reports the existence of a stable complex formed by the probe and the target, is selected from one of the following groups: a conjugate, a branched detection system, a chromophore, a fluorophore, radioisotope, an enzyme, a hapten or a luminescent compound.

The method described in the present invention comprises contact of a sample with at least one PNA probe with a similar sequence to that previously described. Consequently, the analysis is based on a single test with a definitive result in opposition to the conventional methods for the detection of *E*. *coli* 0157 that are based on phenotypic features and require several days to yield the result.

It is still the object of the present invention a kit suitable for carrying out the test to detect specifically, this is, to find, identify or measure the *E. coli* 0157 present in biological samples. The kit comprises the PNA probe and other selected reagents or compounds needed to perform *in situ* hybridization tests.

In a more preferable realization, the kit suitable for performing the assay for the detection, identification or quantification of *E. coli* O157 comprises additionally a fixation, hybridization and wash solution.

Preferably, the method intends to be a diagnostic adjuvant for therapeutic decision and quality control. Thus, the implementation of this method for *E. coli* 0157 identification will allow the adequate clinical treatment to the bacterium and the early identification of the contamination source.

The PNA probes can be applied directly on the sample prepared on a slide, since the application of these probes doesn't involve the use of reagents or enzymes for the permeabilization of the cellular membranes before the hybridization. However, some of the compounds that are frequently used in hybridization are required. Therefore, the probes are normally included in more user-friendly kits. If the desired approach involves the PNA-FISH analysis by flow cytometry, the probe could be applied to the sample in suspension, using the same hybridization compounds.

### Detailed description of the invention

### I - Definitions

a) As used herein, the term "nucleotide" includes natural and artificial molecules known generally by those who use technology related with nucleic acids, to thereby generate polymers that bind specifically to nucleic acids;
b) When used the term "nucleotide sequence" is the same as referring to a segment of a polymer containing subunits, in this case the nucleotides;
c) The term "target sequence" refers to a nucleotide sequence of *E. coli* 0157 that is intended to be detected in the test, where the portion of nucleotides of the probe is designed to hybridize;
d) The term "PNA probe" refers to a polymer of subunits of PNA which has a nucleotide sequence and is specific to hybridize with a target sequence of the microorganism of interest. PNA molecules are DNA mimics in which the negatively charged sugar-phosphate backbone structure is replaced by an achiral and electrically neutral formed by repeated N - (2-aminoethyl) glycine units;
e) When using the term "detectable fraction", it refers to molecules that can be connected to the probe, to thereby render the probe detectable by an instrument or method;
f) The term "sample" refers to any biological sample that may contain the microorganism or target sequence for detection. The samples could be clinical (e.g. blood, urine, feces, etc.), food (e.g. meat, eggs, infant formulae, milk, etc.) or environmental (e.g. water).

### II - Brief Description of the Drawings

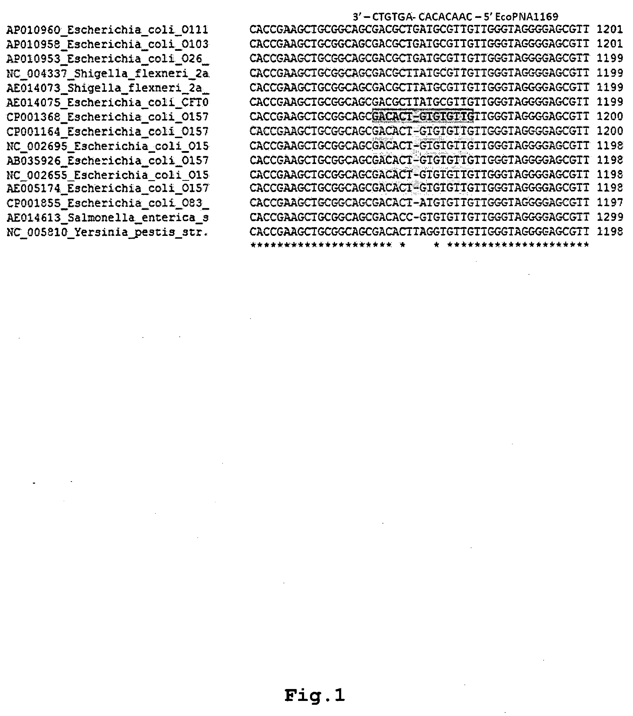

Figure 1 presents the partial alignment of the 23S rDNA sequences for probe selection. The antisense complementary sequence of the EcoPNA1169 probe is shown above the alignment and the polymorphic positions are marked as well.

### III - Description

### PNA probe design:

To identify potentially useful oligonucleotides sequences to use as probe, rDNA 16S and 23S sequences available at the National Center for Biotechnology Information (NCBI) website (http://www.ncbi.nlm.nih.gov/guide/), were chosen. This selection included six *E. coli* O157:H7, five *E*. *coli* non-O157:H7 serotypes and four other strains from related species belonging to the *Enterobacteriaceae* family (Figure 1). Those sequences were aligned using the CluscalW software available at the European Bioinformatics Institute (EMBL-EBI, www.ebi.ac.uk/clustalw/). A conserved region in the 23S rRNA of all 0157:H7 strains of *E. coli* have been identified (figure 1). The criteria for the selection of the final sequence of the probe included: Guanine/Cytosine percentage; the type of secondary structures and hybridization temperature. After a evaluation of those parameters the following sequence was chosen: 5'-CAA CAC ACA GTG TC-3'. This sequence hybridizes between positions 1169 and 1183 of strain TW14359 *E*. *coli* 0157:H7 (accession number CP_001368). The probe was named EcoPNA1169 due to the initial position of the target sequence. Subsequently, the chosen sequence was synthesized and the oligonucleotide was attached, at the N terminus, to the fluorochrome Alexa Fluor594.

### Theoretical evaluation of the PNA probe performance:

After the design of the probe, its performance was evaluated by determine the theoretical values for sensitivity and specificity. These parameters were evaluated with the above mentioned software ProbeCheck available in the ARB Silva database for rRNA. For this theoretical estimation only the good-quality sequences with at least 1900 bp were considered, as well as strains of *E. coli* with assigned serotype. The probe was aligned with a total of 180 344 sequences present in the database for the large RNA subunit (LSU, 23S/28S). The probe was also tested against the database for the small subunit (SSU, 16S/18S) to access the existence of possible cross-hybridization with the 16S rRNA sequences. Specificity was calculated as nECs/(TnECs) x 100, were nECs stands for the number of non-*E*. *coli* 0157:H7 strains not detected by the probe and TnECs is the total number of non-*E*. *coli* 0157:H7 strains examined. Sensitivity was calculated as ECs/(TECs) x 100, where ECs stands for the number of *E*. *coli* 0157:H7 strains detected by the probe and TECs is the total of number of *E*. *coli* 0157:H7 present in the database. Through ProbeCheck program, was possible to found that the EcoPNA1169 probe detect all 80 sequences of *E. coli* 0157:H7 existing in the database, and also 11 other sequences, performing a total of 91 sequences detected (last accessed, July 2012). Therefore, a theoretical specificity and sensitivity of 88 and 100% were obtained, respectively. The 11 non-*E*. *coli* O157:H7 strains detected by the EcoPNA1169 probe, included 3 non-O157 *E. coli,* 7 *Salmonella* spp. and 1 *Cronobacter* spp. strain.

The PNA probe of this invention comprises preferably 14 nucleotides and may be at least 86% identical to the sequence SEQ ID No. 1 - 5'- CAA CAC ACA GTG TC -3', preferably 87%, 88%, 89%, 90%, 91%, 92%, 93%, 94%, 95%, 96%, 97%, 98%, 99%, 100% of similarity to SEQ ID No. 1 - 5'- CAA CAC ACA GTG TC -3'.

Alternatively, this invention also contemplates variations of the nucleotide sequences of the probes. Such variations may include deletions, insertions, among others. For example on of the following sequences:
- SEQ ID No. 2 - 5'- AAC AAC ACA CAG TG -3';
- SEQ ID No. 3 - 5'- AAC ACA CAG TGT CG -3';
- SEQ ID No. 4 - 5'- AAC AAC ACA CAG TGT C -3';
- SEQ ID No. 5 - 5'- CAA CAC ATA GTG TC -3'.

### Detectable fraction of the PNA probe:

Not limited to the following examples, the detectable fraction of PNA probe can include various types of molecules such as dextran conjugates, chromophores, fluorophores, radioisotopes, enzymes, haptens, chemiluminescent compound, among others.

As an example, among the fluorophores class those that are preferable for use are (but not limited to): Alexa series fluorophores, Alexa Fluor series, cyanines, 5 - (and -6) Carboxy-2',7'-dichlorofluorescein, 5-ROX (5-carboxy-X-rhodamine, triethylammonium salt).

### Method:

The present invention presents a method for detecting the presence of *E. coli* O157 using a nucleotide sequence with at least 86% of homology with the region of 14 nucleotides here described - SEQ ID No.1.

The method can include the contact of a sample with the PNA probe described herein with the bacterium target sequence in appropriate hybridization conditions or appropriate *in situ* hybridization conditions (as shown in EXAMPLE 1).

The method can be divided into: sample preparation (which includes the enrichment step, when necessary), fixation, hybridization, wash and visualization of the results (see EXAMPLE 1).

The method can be performed on adhered or suspended cells.

### Protocol optimization:

PNA-FISH methodology involves 4 steps, fixation and permeabilization of the sample; hybridization of the probe, wash of non-hybridized probe and observation on a fluorescence microscope. The following steps are a possible optimization of the hybridization conditions, without being a limitation of this invention:
There are several factors that influence the hybridization of the PNA probes and the target sequence. These include the percentage of formamide (or other denaturing chemical reagent), salt concentration and consequently the ionic strength, ethanol percentage, temperature of hybridization and washing, detergent concentration, pH and others.

To determine the optimal hybridization conditions, with high stringent conditions, it may be necessary to fix the different factors and change each factor individually until a desirable discriminatory degree is achieved.

The closer a target sequence is from another non-target in the sample, the greater the stringency degree needed to define the various factors that influence the hybridization.

In this invention non-target sequences (this is, non-*E*. *coli* O157:H7 sequences) can have only one different nucleotide in comparison to the target sequences, and as such an increased level of discrimination is necessary to avoid non-specific hybridizations.

In order to understand the behavior of EcoPNA1169 probe and infer about the best hybridizations conditions, hybridization and washing temperature were ranged between 53 and 61°C; fixation step using ethanol was ranged between 50 and 80% and finally different hybridization times were tested, 30, 45, 60 and 90 minutes.

After the optimization of all the parameters referred above, the procedure that was found to result in a stronger fluorescent signal was as follows:
Smears of each bacterial culture (about 20 µl) in microscope slides appropriate for fluorescence visualization were prepared. The smears were immerse in 4% (wt/vol) para-formaldehyde (Sigma) followed by 50% (vol/vol) ethanol for 10 min each and allowed air dry. The smears were then covered with 20 µL of hybridization solution containing 10% (wt/vol) dextran sulfate (Sigma), 10 mM NaCl (Sigma), 30% (vol/vol) formamide (Sigma), 0.1% (wt/vol) sodium pyrophosphate (Sigma), 0.2% (wt/vol) polyvinylpyrrolidone (Sigma), 0.2% (wt/vol) Ficoll (Sigma), 5 mM disodium EDTA (Sigma), 0.1% (vol/vol) Triton X-100 (Sigma), 50 mM Tris-HCl (pH 7.5; Sigma) and 200 nM of PNA probe. Samples were covered with coverslipes, placed in moist chambers and incubated for 45 min at 59°C. Subsequently, the coverslipes were removed and the slides were submerged in a prewarmed (59°C) washing solution for 30 minutes, containing 15 mM NaCl (Sigma), 0,1% (vol/vol) Triton X-100 (Sigma) and 5 mM Tris base (pH 10; Sigma). Afterward, the slides were removed from the wash solution and dried at 59 °C in the same incubator for approximately 5 minutes. Before the microscope observation, a drop of non-fluorescent immersion oil (Merck) was placed and covered with a cover slip. The slides were stored in the dark for a maximum of 24h before microscopy.

### Probe experimental specificity and sensitivity evaluation:

In order to evaluate the experimental specificity and sensitivity of the PNA probe, the above described protocol was applied to 53 strains. Those included 18 *E*. *coli* 0157:H7 and 25 non-*E. coli* 0157:H7. The two *E. coli* 0157 strains (CCC-18-12 and CCC-26-12) with uncharacterized "H" antigen, were excluded from the calculations. Additionally were included 8 taxonomically related stains belonging to the same genus and family (*Escherichia, Salmonella, Enterobacter, Shigella and Klebsiella*). The results show that hybridization occurs only with strains of *E. coli* 0157:H7 and therefore the sensitivity and specificity values obtained were both 100%.

**Table 1 - Results of the EcoPNA1169 probe specificity and sensitivity test.**

| **Strain** | **Serotype** | **Isolation origin** | **Verotox in production** | **PNA FISH outcome** |
|---|---|---|---|---|
| *E*. *coli* CECT 4267 | 0157:H7 | Human stool from outbreak of hemorrhagic colitis | stx1, stx2 | + |
| *E. coli* CECT 4782 | 0157:H7 | Human stool from outbreak of hemorrhagic colitis | stx1, stx2 | + |
| *E. coli* CECT 4783 | 0157:H7 | Raw hamburger meat implicated in hemorrhagic colitis outbreak | stx1, stx2 | + |
| *E. coli* CECT 5947 | 0157:H7 | - | Gene stx2 has been replaced | + |
| *E. a coli* NCTC 12900 | 0157:H7 | - | NT | + |
| *E. coli* CCC-1-12* | O157:H7 | Faecal swab | stx2 | + |
| *E. coli* CCC-5-12* | 0157:H7 | Faecal swab | stx2 | + |
| *E. coli* CCC-7-12* | 0157:H7 | Faecal swab | stx2 | + |
| *E. coli* CCC-10-12* | 0157:H7 | Faecal swab | stx2 | + |
| *E. coli* CCC-11-12* | O157:H7 | Milk filter | stx2 | + |
| *E. coli* CCC-12-12* | 0157:H7 | Milk filter | stx2 | + |
| *E. coli* CCC-13-12* | 0157:H7 | Milk filter | stx2 | + |
| *E. coli* CCC-14-12* | 0157:H7 | Bovine milk filter | stx2 | + |
| *E. coli* CCC-15-12* | 0157:H7 | Bovine milk filter | stx2 | + |
| *E. coli* CCC-16-12* | 0157:H7 | Caprine milk filter | stx2 | + |
| *E. coli* CCC-18-12* | 0157 | Bovine milk filter | stx2 | + |
| *E. coli* CCC-23-12* | 0157:H7 | Milk filter | NT | + |
| *E. coli* CCC-24-12* | 0157:H7 | Milk filter | NT | + |
| *E. coli* CCC-25-12* | 0157:H7 | Milk filter | NT | + |
| *E. coli* CCC-26-12* | 0157 | Milk filter | NT | + |
| *E. coli* CECT 352 | 0127a:K63(B8):H - | - | EPEC | - |
| *E*. *coli* CECT 504 | 0141:K85(B):H4 | Swine oedema, | ND | - |
| *E. coli* CECT 515T | O1:K1(L1):H7 | Human urine - cystitis | ND | - |
| *E. coli* CECT 533 | 0103:K-:H- | - | ND | - |
| *E. coli* CECT 727 | 0111:K58(B4):H- | Infantile gastroenteritis | EPEC | - |
| *E. coli* CECT 730 | O55:K59(B5):H- | - | ND | - |
| *E. coli* CECT 736 | O28a,28c:K73 (B1 8):H- | Faeces | ND | - |
| *E. coli* CECT 740 | O125a,125b:K70 ( B15):H19 | Gastroenteritis | ND | - |
| *E. coli* CECT 744 | 0158:K-:h23 | Faeces of infant with diarrhoea | ND | - |
| *E. coli* CECT 832 | O111:K58 (B4) : H- | Infantile gastroenteritis | ND | - |
| *E. coli* .CECT 4537 | O10 : K5 (L5) : H4 | Human peritonitis | ND | - |
| *E. coli* CECT 4555 | 097:K-:H- | - | ND | - |
| *E. coli* CCC-2-12* | 0103 | Faecal swab | NT | - |
| *E. coli* CCC-3-12* | 026 | Faecal swab | stx1, stx2 | - |
| *E. coli* CCC-4-12* | 026 | Faecal swab | NT | - |
| *E. coli* CCC-8-12* | O26 | Faecal swab | NT | - |
| *E. coli* CCC-9-12* | O26 | Faecal swab | NT | - |
| *E. coli* CCC-19-12* | O26 | Caprine milk filter | stx1 | - |
| *E. coli* CCC-20-12* | O26 | Caprine milk filter | stx1 | - |
| *E. coli* CCC-21-12* | O26 | Bovine milk filter | stx1 | - |
| *E. coli* CCC-22-12* | O26 | Bovine milk filter | stx1 | - |
| *Escherichi a coli* CECT 434 | O6 | Clinical Isolate | ND | - |
| *Escherichi a coli* N9* | ND | Porcine faeces | ND | - |
| *Escherichi a coli* N5* | ND | Bovine faeces | ND | - |
| *Escherichi a coli* ATCC 29425 (K12) | OR:H48:K- | - | ND | - |
| *Escherichi a hermanii* ATCC 33650 | NR | Human isolate | - | - |
| *Escherichi a vulneris* ATCC 29943 | NR | Human wound | - | - |
| *Shigella boydii* ATCC 9207 | NR | - | ND | - |
| *Salmonella enterica enterica s* orovar Typhimurium NCTC 12416 | NR | - | - | - |
| *Salmonella enterica enterica s* orovar Typhi SGSC 3036 | NR | - | - | - |
| *Salmonella entritidis* SGSC 2476 | NR | - | - | - |
| *Enterobact* | NR | Child's | - | - |
| *er sakasaki* CECT 858 | | throat | | |
| *Klebsiella pneumoniae* ATCC 11296 | NR | - | - | - |

| | | | | |
|---|---|---|---|---|
| NT - non-toxigeninc *E. coli;* EPEC - enteropathogenic *E. coli* (epidemiologically implicated as pathogens, but virulence mechanism is not related to the excretion of enterotoxins); ND - Not determined; NR - non-relevant information for the present study; * - Isolates; SGSC - *Salmonella* Genetic Stock Centre; ATCC - American Type Culture Collection; NCTC - National Collection of Type Cultures; CECT - Spanish Type Culture Collection. | | | | |

### Enrichment:

The samples to be analyzed can be obtained from food, water, feces, blood among others.

The samples containing *E*. *coli* 0157:H7 usually present low contamination levels. Because of this, an enrichment step that facilitates the detection process is recommended. This enrichment step can be performed using several types of culture media, from complex rich media (such buffered peptone water and trypticase soy broth) to selective media, such as: GN (Gram Negative) broth, R & F® Enrichment Broth (R&F-EB) or *E. coli* (EC) broth (Vimont et *al.,* 2006).

Trypticase Soy Broth (TSB) is the most often used enrichment medium. Antibiotics such as novobiocin (most common), cefixime, cefsulodin or vancomycin and other selective compounds (such as bile salts - inhibit non-*Enterobacteriaceae* strains); are often added to this medium to promote a selective enrichment. These broths are then incubated for a period that usually varies between 16 and 24 hours (overnight growth) at 35 to 42°C. However, the data concerning the efficiency of enrichment protocols are very few and differ from study to study.

The incubation temperature does not seem to be related to the serotype sought (Vimont *et al.,* 2006.), but some authors have demonstrated that *E. coli* 0157:H7 strains generally have an average optimal temperature around 40°C. Actually the ISO recommended for detection of *E. coli* 0157 in food samples (ISO 16654:2001 - Microbiology of food and animal feeding stuffs - Horizontal method for the detection of *Escherichia coli* O157) includes a pre-enrichment in the broth mTSB at 41.5°C.

In order to evaluate the influence of the enrichment medium and incubation temperature on the detection limit of the PNA-FISH method, two different broths at 37 and 41.5°C were tested. For this test the chosen broths were the selective medium mTSB with novobiocin (mTSB + N), that is currently recommended by the International Organization for Standardization (ISO 16654:2001) and the BPW (peptonated buffered water), a non-selective broth widely used in enrichment protocols of several pathogens, which has also been applied to the detection of *E*. *coli* O157: H7.

The PNA probe described in this invention was tested in two different types of samples: ground beef and unpasteurized milk (two commonly associated matrices with infections by *E*. *coli* O157:H7) artificially contaminated with concentrations ranging from 0.01 to 100 CFU/g or 25 ml of food sample. Two strains of *E. coli* 0157:H7 were used (CCC-5-12 and CECT 4267) for inoculation and the samples were simultaneously analyzed by ISO 16654:2001. As seen in Table 2, the mTSB gave the best detection limit, while the use of higher temperatures (41.5°C) do not seem to improve the detection rate of *E. coli* 0157. The good performance of mTSB may be related to the selective nature of the broth, that includes novobiocin and bile salts, which partially inhibit the growth of existing microflora in food. However, after determining the growth rate of both strains of *E*. *coli* in BPW and mTSB, was observed that the composition of the broth (and not the selective factors) is the determining factor for bacterial growth. Growth rates of ≈ 0.5 h⁻¹ (41.5°C) and 0.4 h⁻¹ (37°C) were observed for the strains cultivated in mTSB, while those grown in BPW had values of 0.07 h⁻¹ for both temperatures. To better quantify the performance of each enrichment method, the sensitivity and specificity for both media, mTSB and BPW at 37°C were determined based on the results presented in Table 3. The obtained specificity values were 100% for both broths (confidence interval [CI] 95% from 69.87 to 100), while the sensitivity values were 94.44% (95% CI, 70.63 to 99.71) to mTSB and 55.55% (95% CI, 31.35 to 77.59) for BPW. Although the best performance has been found to mTSB + N, it shall be possible to standardize the enrichment step to allow the simultaneous detection of different food pathogens.

Another important feature to consider when optimizing FISH protocols is that some dietary components may present a strong autofluorescence signal, and thus interfere with the detection of the bacterium. In order to eliminate/reduce this phenomenon, ark additional step can be implemented before the hybridization procedure. Two different approaches were tested: one centrifugation step (to remove food particles) and using a detergent (Triton X-100, 1%) to emulsify the lipid compounds. Both steps have decreased autofluorescence signal, but the detergent showed a more significant reduction and also seemed to improve the fluorescence signal. This could arise from the detergent ability to help in the cellular permeabilization.

Regarding the detection time of the assay, the PNA-FISH method implementation can save at least 2 days in the detection of *E*. *coli* 0157:H7, when compared with the traditional protocol.

In conclusion, we found that the here described PNA-WISH method has a detection limit of 1 CFU per 25 g of food after an overnight enrichment step on mTSB. The comparison with the traditional culture method showed a value of 100% specificity and 94% sensitivity. Finally, it was observed that the use of selective compounds and higher temperature lead to a narrow improvement regarding the detection limit of the method.

**Table 2 - PNA FISH results obtained in the detection of E. coli 0157:H7 in different food matrices inoculated with bacterial concentrations between 0.01 and 25 g or 100 CFU per ml of food. The results include 3 independent experiments.**

| **Concentration** | **Ground beef** | | | | **Unpasteurized milk** | |
|---|---|---|---|---|---|---|
| **(CFU/25g or ml)** | 37°C | | 41,5°C | | 37°C | 41,5°C |
| | mTSB+N | BPW | mTBS+N | BPW | mTBS+N | mTBS+N |
| 100 | + (6/6)^{a} | + (6/6)^{a} | + (6/6)^{a} | + (6/6)^{a} | + (6/6)^{a} | + (6/6)^{a} |
| 10 | + (6/6)^{a} | + (4/6)^{a} | + (6/6)^{a} | + (2/6)^{a} | +(6/6) ^{a} | + (6/6)^{a} |
| 1 | + (5/6)^{a} | - (0/6)^{a} | + (5/6)^{a} | - (0/6)^{a} | + (6/6) ^{a} | + (6/6)^{a} |
| 0.1 | - (6/6)^{b} | - (6/6)^{b} | - (6/6)^{b} | (6/6)^{b} | - (6/6)^{b} | - (6/6)^{b} |
| 0.01 | - (6/6)^{b} | - (6/6)^{b} | - (6/6)^{b} | - (6/6)^{b} | - (6/6)^{b} | - (6/6)^{b} |

| | | | | | | |
|---|---|---|---|---|---|---|
| a - Samples that tested positive by PNA FISH/Total positive samples determined by culture method; b - samples that tested negative by PNA-FISH/Total negative samples determined by culture. | | | | | | |

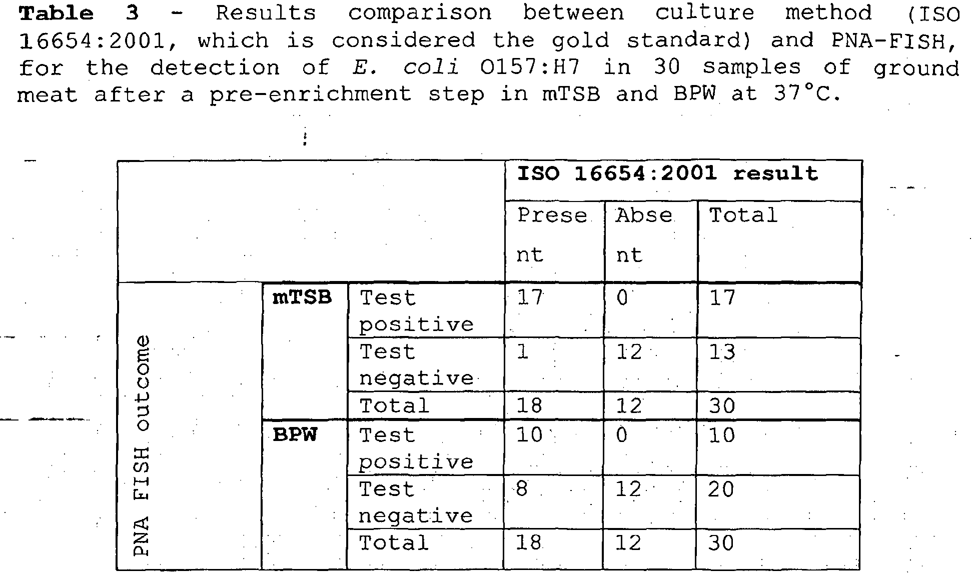

### Visualization of the results:

This step can be performed in any epifluorescence microscope with a filter sensitive to fluorophore used. Other filters present in the microscope, which are not able to detect the fluorescent signal of the probe, were used to confirm the absence of autofluorescence.

### Kit:

The present invention also refers to a kit that allows testing for the presence of *E*. *coli* O157:H7.

The kit of the present invention comprises a PNA probe at least 86% identical to SEQ ID No. 1 and another reagents or compositions that are selected to perform the test.

The PNA probe, its characteristics, the methods and kit of this invention are suitable for the analysis of nucleic acids sequences present, or not, internally in the target organism. As such, this invention can be used for both, the organism or nucleic acids extracted or derived from the organism of interest, implying that the source of the target sequence is not a limitation on this invention.

The following examples illustrate different situations and several steps for implementing the invention, are preferred embodiments of the present invention, without intending to limit any of them:

### EXEMPLE 1: Detection of E. coli 0157:H7 serotype in different samples (clinical, food or environmental samples)

### Sequence:

SEQ ID No. 1 - 5'- CAA CAC ACA GTG TC -3 (coupled to Alexa Fluor 594).

### Sample preparation:

The food, clinical or environmental samples were previously subjected to an enrichment step before application of the PNA probe. This happens because usually *E. coli* 0157:H7 presents low contamination levels. This enrichment step can be performed using the medium recommended for the conventional analysis method used for each sample. In the case of food, animal feed, feces or water, mTSB was used. Samples were then incubated for 18 - 22 hours at 37°C, at 120 rpm. Following the enrichment step, 15 µl of the culture medium were mixed with 15 µl of Triton X-100 (1%) directly in the microscope slide suitable for fluorescence. The slide was placed for approximately 5 minutes in an incubator oven at 59°C or left to air dry.

### Fixation:

For preventing the loss of 23S rRNA during the hybridization process, the samples were immersed in a solution of 4% paraformaldehyde (wt/vol) and 50% ethanol (vol/vol) for 10 minutes each.

### Hybridization:

After fixation, samples were then covered with a drop of hybridization solution containing: 10% (wt/vol) dextran sulfate (Sigma); 10 mM NaCl (Sigma); 30% (vol/vol) formamide (Sigma); 0.1% (wt/vol) sodium pyrophosphate (Sigma); 0.2% (wt/vol) polyvinylpirrolidone (Sigma); 0.2% (wt/vol) Ficol (Sigma); 5 mM disodium EDTA (Sigma); 0.1% (vol/vol) Triton X-100 (Sigma); 50 mM Tris-HCl (pH 7.5; Sigma); and 200nM of PNA probe. The samples were covered with coverslips (to assure an homogeneous spreading of the probe) placed in small wet boxes (to prevent the evaporation of the hybridization solution) protected from light and incubated for 45 minutes at 59°C.

### Wash:

After the hybridization time, the coverslips were removed and the slides were immersed in a wash solution pre-warmed at 59°C containing 5 mM Tris Base (pH 10), 15 mM NaCl and 1% (vol/vol) Triton X-100. The samples were then placed in an oven at the hybridization temperature for 30 minutes. Subsequently, the slides were removed from the wash solution and dried at 59°C, in the same incubator, for approximately 5 minutes. Before the microscope visualization, a drop of non-fluorescent immersion oil (Merck) was placed and covered with a coverslip. The slides were kept in the dark for a maximum period of 24 hours before microscopy.

### Results:

The results were obtained through the observation in a fluorescence microscope with a filter capable of detecting the fluorochrome Alexa Fluor 594 bonded to the PNA detecting probe.

### EXEMPLE 2: Salmonella and E. coli O157:H7 detection in feces.

This example illustrates the possibility of using the probe for *E*. *coli* 0157:H7 together with the probe for *Salmonella* spp. previous developed in Almeida et *al.,* 2010. Both these two bacteria are a common cause of gastroenteritis and as such, a fast detection in feces is of the most importance. The use of a "multiplex" approach (simultaneous use of multiple probes) can simplify and expedite the detection procedure. These two probes present very similar hybridization temperatures and can be used easily in a multiplex assay.

### Sequences:

SEQ ID No. 1 - 5'- CAA CAC ACA GTG TC -3' (coupled to Alexa Fluor 594).

Sequence from Almeida *et al.,* 2010 paper: 5'- AGG AGC TTC GCT TGC -3' (coupled to Alexa Fluor 448).

### Sample preparation:

25g of feces were mixture with 225 ml of mTSB. Different amount of sample can be used, but maintaining the 1/10 (wt/ vol) ratio. Samples were then incubated for 18 - 22 hours at 37°C, at 120 rpm. Following the enrichment step, 15 µl of the culture medium were mixed directly in a microscope slide suitable for fluorescence with 15 µl of 1% (vol/vol) Triton X-100 to minimize the interference from autofluorescent particles. The slide was placed for approximately 5 minutes in an incubator oven at 59°C or left to air dry.

### Hybridization:

Hybridization was performed as previously described in Example 1 with a small change. The hybridization solution contained two probes: PNA probe for the detection of *Salmonella* and PNA probe for the detection of *E. coli* 0157:H7, each at a concentration of 200nM.

### Washing:

Washing was carried out according to the procedure described in Example 1.

### Results:

The results were obtained by observation under a fluorescence microscope with the appropriate filters for the detection of Alexa Fluor 594 and 488 fluorochrome connected to the PNA probes.

### References:

Gyles CL: Shiga toxin-producing Escherichia coli: an overview. J Anim Sci 2007, 85(13 Suppl):E45-62.
Pennington H: Escherichia coli 0157. Lancet 2010, 376(9750):1428-1435.
Robinson AL, McKillip J: Biology of Escherichia coli O157:H7 in human health and food safety with emphasis on sublethal injury and detection. In: Current Research, Technology and Education Topics in Applied Microbiology and Microbial Biotechnology. Edited by Mendez-Vilas A: Formatex; 2010: 1096-1105.
Raji MA, Jiwa SF, Minga MU, Gwakisa PS: Escherichia coli 0157: H7 reservoir, transmission, diagnosis and the African situation: a review. East Afr Med J 2003, 80(5):271-276.
Cerqueira L, Azevedo NF, Almeida C, Jardim T, Keevil CW, Vieira MJ: DNA mimics for the rapid identification of microorganisms by fluorescence in situ hybridization (FISH). Int J Mol Sci 2008, 9(10):1944-1960.
Almeida C, Azevedo NF, Fernandes RM, Keevil CW, Vieira MJ: Fluorescence in situ hybridization method using a peptide nucleic acid probe for identification of Salmonella spp. in a broad spectrum of samples. Appl Environ Microbiol 2010, 76(13):4476-4485.
Vimont A, Vernozy-Rozand C, Delignette-Muller ML: Isolation of E. coli 0157:H7 and non-O157 STEC in different matrices: review of the most commonly used enrichment protocols. Lett Appl Microbiol 2006, 42(2):102-108.

## Claims

1. PNA probe for the detection and/or quantification of *E. coli* 0157:H7 serotype **characterized in that** it comprises at least one sequence with at least 86% similarity to SEQ ID No. 1 5'- CAA CAC ACA GTG TC -3'.

2. PNA probe, according to claim 1, **characterized in that** it comprises at least one sequence SEG ID No. 1 and considering variations in the nucleotide sequences of the probes for instance in the following sequences SEQ ID No. 2, SEQ ID No. 3, SEQ ID No. 4, SEQ ID No. 5.

3. PNA probe, according to claim 1, **characterized in that** it is capable of detecting the target sequence in rRNA, rDNA or the sequences complementary to *E. coli* 0157:H7 rRNA.

4. PNA probe, according to claim 1, **characterized in that** it additionally comprises one sequence with at least 86% similarity to SEQ ID No 5.

5. PNA probe, according to any of the claims 1 - 4, **characterized in that** it is connected to at least one type of detectable fraction.

6. PNA probe, according to claim 5, **characterized in that** the type of detectable fraction of the probe is selected from one of the following groups: a conjugate, a branched detection system, a chromophore, a fluorophore, radioisotope, an enzyme, a hapten or a luminescent compound.

7. PNA probe, according to claim 6, **characterized in that** the fluorophore group is at least one of the following: fluorophores of the Alexa series, Alexa Fluor series, cyanines, 5-(and -6) Carboxy-2',7'-dichlorofluorescein, the 5-ROX (5-carboxy-X-rhodamine, triethylammonium salt).

8. Kit for detecting *E. coli* 0157:H7, **characterized in that** it comprises at least one of the probes described in any of the claims 1 to 7.

9. Kit, according to claim 8, **characterized in that** it further comprises at least one of the following solutions: one fixation solution, one hybridization solution and one washing solution.

10. Kit, according to claim 9, **characterized in that** the fixation solution comprises paraformaldehyde and ethanol, namely 2-8% (wt/vol) of paraformaldehyde and 25-90% (vol/vol) of ethanol.

11. Kit, according to claim 9, **characterized in that** the hybridization solution comprises formamide.

12. A method for the specific detection of *E. coli* 0157:H7, **characterized in that** it uses the PNA probes described in claims 1 to 7 and it comprises the following steps:
a. PNA probe contact with the biological samples;
b. PNA probe hybridization with the target sequence of the microorganisms present in the referred samples;
c. Hybridization detection as indication of the referred detection and quantification of the referred samples.

13. Method, according to claim 12, **characterized in that** the biological sample is derived from food, feces, blood, air, water or biopsies.

14. Method, according to claim 12, **characterized in that** the hybridization occurs by fluorescence.

15. Use of PNA probes, as described in any one of claims 1 to 7, **characterized in that** it is applied in a methodology for detecting *E. coli* 0157:H7 in biological samples.

16. Use of the kit, as described in any of claims 8 to 11, **characterized in that** it is applied in the detection of *E*. *coli* O157:H7 in biological samples.
